# FASCICULE DE BREVET EUROPEEN

(11) **EP 2 462 901 B1**
(45) Date de publication et mention de la délivrance du brevet: **05.08.2015**
(21) Numéro de dépôt: 11354074.4
(22) Date de dépôt: 06.12.2011
(51) Int. Cl.: A61F 2/64, A61F 2/60, A61F 2/50

(54) **Prothèse pour membre inférieur**
Prothese für untere Gliedmaßen
Lower limb prosthesis

(30) Priorité: 09.12.2010 FR 1004800
(43) Date de publication de la demande: 13.06.2012
(73) Titulaire: Otto Bock HealthCare GmbH, 37115 Duderstadt (DE)
(72) Inventeur: Chabloz, Pierre, 38450 Saint Georges de Commiers (FR)
(74) Mandataire: Lins, Edgar

(56) Documents cités:
- EP-A1- 1 598 573
- EP-A2- 1 417 942
- US-A1- 2007 050 044
- US-A1- 2007 162 152

## Description

### Domaine technique de l'invention

L'invention est relative à une prothèse pour membre inférieur comportant :
- un organe de réception d'un moignon de cuisse monté sur un élément de support,
- une jambe montée sur ledit élément de support par une liaison pivot formant une articulation de genou pour permettre une flexion ou une extension de la jambe par rapport à l'organe de réception,
- un système d'amortissement pour amortir les efforts de flexion ou d'extension.

### État de la technique

Il a déjà été proposé à des personnes amputées du membre inférieur entre le genou et la hanche de retrouver la possibilité de skier.

Dans une première approche, les personnes amputées skiaient sur une seule jambe, et étaient équipées de béquilles munies de petits skis à leurs extrémités pour assurer l'équilibre.

Dans une seconde approche, il a été proposé de redonner la possibilité aux personnes amputées de skier sur deux membres inférieurs avec un matériel prothétique réalisant des fonctions proches de celles d'un vrai membre inférieur dans des conditions particulières, comme la position en triple flexion (hanche, genou, cheville) avec un amortissement permettant d'absorber le relief d'une piste de ski.

Ainsi, il existe des prothèses articulées pour membre inférieur comportant un organe de réception d'un moignon de cuisse d'un patient. L'organe peut ensuite être monté sur une jambe par une liaison pivot dont l'axe forme une articulation de genou pour permettre une flexion ou une extension de la jambe par rapport à l'organe. Un vérin permet d'amortir les efforts de flexion ou d'extension.

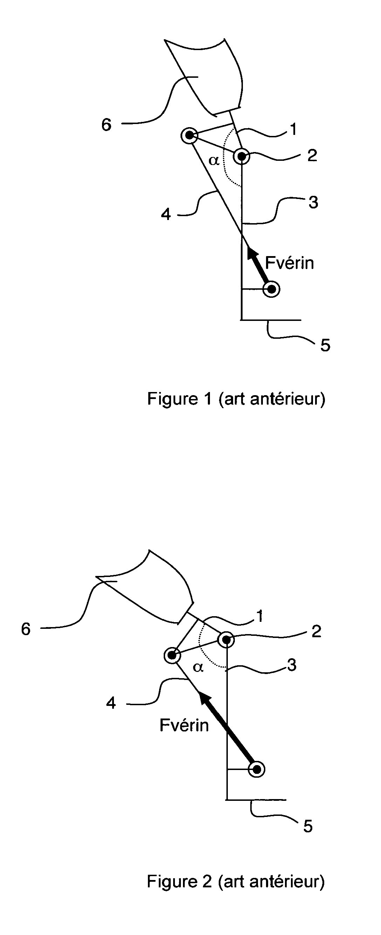

Sur l'exemple des figures 1 et 2, un élément de support 1, sur lequel l'organe de réception 6 est monté, est lui-même monté sur la jambe 3 par une liaison pivot 2 de sorte à former une articulation de genou. Le vérin 4 est monté d'une part sur une partie avant de la jambe 3 (partie en regard d'un pied 5), et d'autre part sur une partie arrière (zone au niveau de laquelle l'angle α se referme en cas de flexion) de l'élément de support 1.

La figure 1 illustre la position de la prothèse en flexion avec un angle α de 155 degrés entre la jambe 3 et l'organe de réception 6 au niveau de la flexion. La figure 2 illustre un angle α de 110 degrés. Avec l'angle α à 155 degrés, la force exercée par le vérin 4 Fverin est d'environ 2500 Newton alors qu'à 110 degrés la force exercée par le même vérin 4 Fverin est d'environ 5100N. En fait, plus la prothèse sera fléchie plus le vérin aura du mal à contrer la flexion, l'utilisateur se retrouve alors sans système d'amortissement pouvant entraîner la rupture au niveau de l'articulation. De plus, le confort d'utilisation de la prothèse s'en retrouve très dégradé.

Le document EP 1 598 573 A1 concerne un amortisseur de mouvements réglable comportant un piston d'amortissement qui délimite deux chambres d'amortissement l'une par rapport à l'autre dans un cylindre d'amortissement. Un clapet d'amortissement est agencé dans un conduit de liaison entre les deux chambres d'amortissement. Le clapet d'amortissement peut être commandé vis-à-vis du débit par un organe de commande. L'organe de commande prévoit un ensemble de piston de poussée qui est rempli d'un liquide magnétorhéologique. L'ensemble de piston de poussée est couplé en coopération de forces au piston d'amortissement par un piston de poussée.

Le document US 2007/050044 A1 concerne une prothèse de jambe comportant une articulation de genou régénérative à commande électronique. Selon un mode de réalisation, l'articulation de genou de la prothèse peut être réalisée uniquement de façon passive pour générer de l'énergie électrique, et selon un autre mode de réalisation, l'articulation de genou peut être réalisée de façon active, ce qui signifie qu'elle assiste à la marche ou bien la commande complètement et qu'elle génère en outre de l'énergie électrique. Un amortisseur hydraulique peut être fixé ensemble avec le générateur dans un cadre. Les points de fixation supérieurs du générateur et de l'amortisseur sont montés sur un support mobile autour d'un axe de pivotement. Le document EP 1 417 942 A2 décrit une prothèse de genou comportant un cadre et un dispositif d'amortissement fixé dans le cadre. À l'extrémité proximale du cadre est prévu un levier dont une extrémité comprend le dispositif d'amortissement et dont l'autre extrémité comprend un dispositif de réglage pour régler le mouvement de pivotement.

Le document US 2007/0162152 A1 qui divulgue les caractéristiques du préambule de la revendication 1 annexée concerne des articulations artificielles comprenant des actionneurs qui agissent en sens opposés l'un à l'autre. Deux actionneurs peuvent être agencés à des extrémités opposées d'une poutre transversale qui est fixée en pivotement sur une partie inférieure d'articulation.

### Objet de l'invention

L'objet de l'invention vise à réaliser une prothèse plus solide et plus proche du comportement d'une jambe valide.

On tend vers cet objet par les revendications annexées et notamment en ce que le système d'amortissement est monté d'une part sur l'axe de la liaison pivot, et d'autre part sur une première extrémité d'une bascule montée à pivotement sur la jambe, et en ce qu'une biellette est montée d'une part sur une seconde extrémité de la bascule et d'autre part sur l'élément de support.

Un tel montage, permet aussi une meilleure stabilisation d'un utilisateur de la prothèse faisant du ski.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
Les figures 1 et 2 illustrent schématiquement des dispositifs de l'art antérieur dans des positions distinctes.
La figure 3 illustre schématiquement un dispositif particulier selon l'invention.
La figure 4 illustre une vue en trois dimensions de la jambe et de l'élément de support en position de flexion.
La figure 5 illustre une vue en trois dimensions de la jambe et de l'élément de support en position d'extension.
La figure 6 illustre une vue de la jambe et de l'élément de support sans le corps principal de la jambe.
La figure 7 illustre une vue différente de la figure 6.
La figure 8 illustre une vue en trois dimensions dans laquelle la biellette est désengagée.
La figure 9 illustre la biellette utilisée selon un mode de réalisation particulier.
La figure 10 illustre un mode de réalisation particulier d'un membre inférieur.

### Description de modes préférentiels de réalisation

La prothèse décrite ci-après diffère de la prothèse selon l'art antérieur en ce que son assemblage particulier permet de limiter les efforts du système d'amortissement.

Par membre inférieur d'une personne, on entend une cuisse, un genou et un pied. La cuisse est la partie située entre la hanche et le genou, la jambe est la partie située entre le genou et le cou-de-pied du pied.

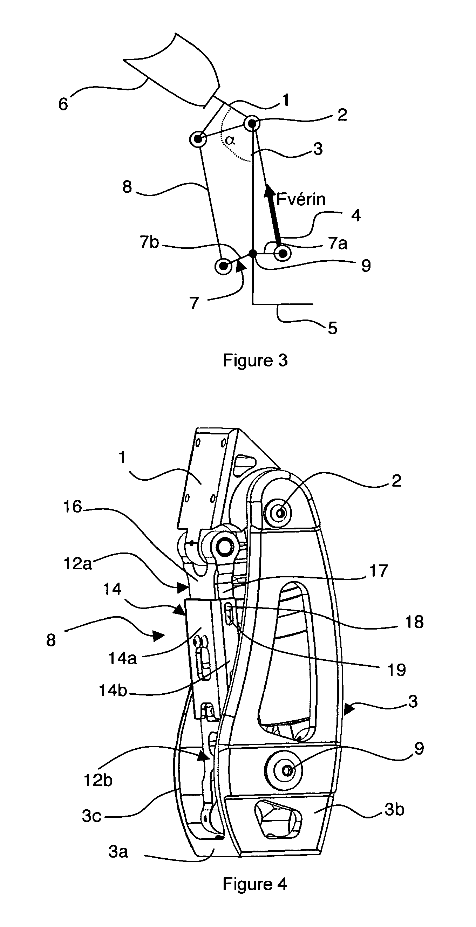

La figure 3 illustre schématiquement une prothèse pour membre inférieur comportant un organe de réception 6 d'un moignon de cuisse monté sur un élément de support 1. Une jambe 3 est montée sur ledit élément de support par une liaison pivot 2 formant une articulation de genou pour permettre une flexion ou une extension de la jambe 3 par rapport à l'organe de réception (selon l'angle α à la figure 3). Un système d'amortissement 4, permettant d'amortir les efforts de flexion ou d'extension, est monté d'une part sur l'axe de la liaison pivot 2, et d'autre part sur une première extrémité d'une bascule 7 montée à pivotement sur la jambe 3. Une biellette 8 est montée d'une part sur une seconde extrémité de la bascule 7 et d'autre part sur l'élément de support 1 de sorte à transmettre le mouvement de pivotement de l'élément de support autour de l'axe de la liaison pivot 2 à la bascule pour plus ou moins solliciter le système d'amortissement.

La biellette 8 permet, lorsque l'élément de support 1 pivote autour de l'axe de la liaison pivot 2, de transmettre le pivotement à la bascule, répercutant ainsi le mouvement au système d'amortissement 4 en diminuant, ou augmentant, la distance séparant le point de montage du système d'amortissement sur la bascule 7 du point de montage au niveau de l'axe de la liaison pivot 2. Autrement dit plus l'angle α sera petit, plus le système d'amortissement sera comprimé, c'est-à-dire qu'en cas de flexion la distance séparant le point de montage dudit système 4 sur la bascule se rapprochera de l'axe de la liaison pivot 2. Bien entendu, avantageusement, les points de montage respectif du système d'amortissement 4 et de la biellette 8 au niveau de la bascule sont réalisés à des extrémités de la bascule 7 disposées de part et d'autre de l'axe de basculement 9 de ladite bascule 7. En outre dans la mise en oeuvre particulière, le point de montage de la biellette 8 au niveau de l'élément de support 1 est situé du même côté que le point de montage de la biellette 8 sur la bascule 7.

Autrement dit, la bascule 7 peut comporter de part et d'autre de son axe de basculement 9 deux branches 7a, 7b. Une première branche 7a étant orientée vers une première face appelée face avant de la prothèse (la face avant est en fait la face de la prothèse en regard d'un pied 5). Une seconde branche 7b est orientée vers une seconde face, opposée à la première face, et appelée face arrière de la prothèse (la face arrière de la prothèse correspond à la face postérieure d'un membre inférieur, c'est-à-dire la face qui va permettre le rapprochement de l'organe de réception 6 de la jambe 3 par flexion). Sur l'exemple particulier de la figure 3, le système d'amortissement 4 est monté sur la première branche 7a de la bascule 7 à une extrémité de la première branche 7a proximale de la face avant de la prothèse. La biellette 8 est montée sur la seconde branche 7b de la bascule 7, à une extrémité de la bascule 7 proximale de la face arrière de la prothèse, et le montage de la biellette 8 au niveau de l'élément de support 1 est réalisé au niveau de la face arrière de la prothèse.

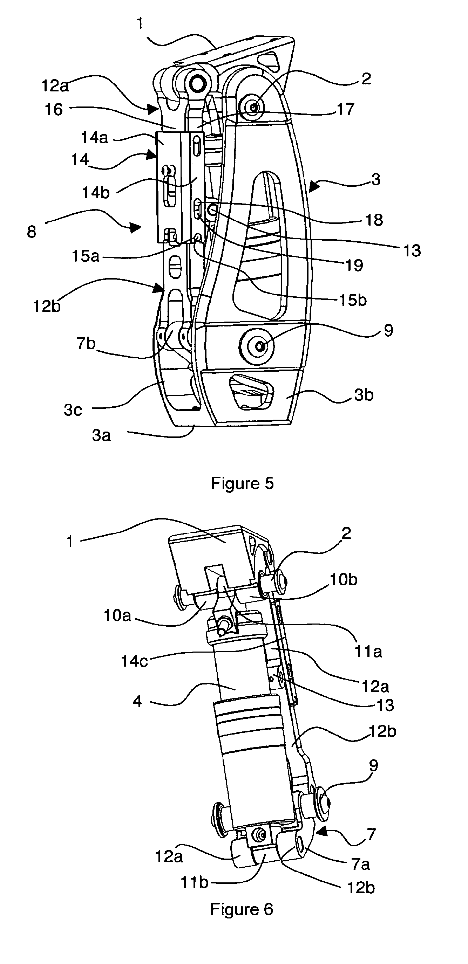

Les figures 4 et 5 illustrent un mode particulier de réalisation. Selon ce mode particulier, la jambe 3 peut avoir une pièce principale monobloc comportant une base 3a reliant deux montants 3b, 3c sensiblement parallèles entre eux. À l'extrémité de la jambe 3, distale de la base 3a, un axe peut être monté de sorte à relier, de préférence perpendiculairement, les deux montants 3a, 3b. Cet axe peut être fixe par rapport aux montants 3b, 3c. Sur cet axe, destiné à former la liaison pivot 2 évoquée ci-dessus, l'élément de support 1 est monté à pivotement.

L'élément de support 1 peut être une pièce, par exemple monobloc, comprenant une première zone de montage destinée à recevoir l'axe de la liaison pivot 2, et une seconde zone de montage destinée à recevoir un axe pour le montage d'une extrémité de la biellette 8. Comme illustré à la figure 6 sur laquelle le corps principal de la jambe 3 a été retiré pour des raisons de clarté, la première zone de montage peut comporter deux tronçons 10a, 10b comprenant chacun un alésage, les alésages desdits tronçons 10a, 10b étant coaxiaux. Les deux tronçons 10a, 10b sont séparés par un espace de vide permettant de recevoir, entre les deux tronçons 10a, 10b, une extrémité du système d'amortissement 4 munie d'une première pièce de montage 11a comportant un alésage destiné à recevoir l'axe de la liaison pivot 2. Ainsi, en position montée, l'axe de la liaison pivot 2 traverse les deux alésages des tronçons 10a, 10b de l'élément de support 1, et l'alésage de la première pièce de montage 11 a.

Sur les figures 4 et 5, l'axe de la liaison pivot 2 de l'articulation est fixé au niveau des deux montants 3b, 3c la jambe 3, et l'élément de support 1 et le système d'amortissement 4 sont montés à pivotement/rotation sur l'axe de la liaison pivot 2 entre les deux montants 3b, 3c.

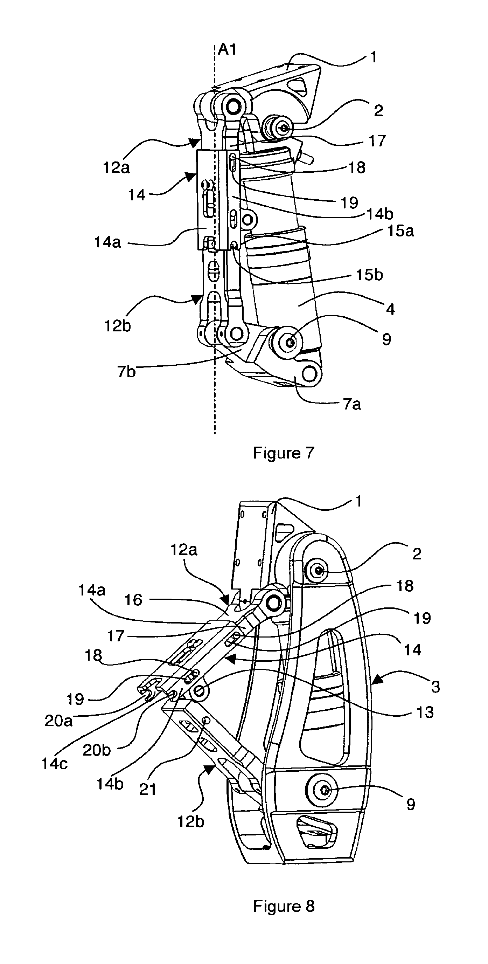

Entre les deux montants 3b, 3c et, de préférence, de manière proximale à la base 3a, la bascule 7 peut être solidarisée auxdits deux montant 3b, 3c par l'axe de basculement 9 (figures 4 à 6). La bascule 7 comporte alors deux extrémités distales formées par les branches 7a, 7b visibles à la figure 7, une première extrémité étant orientée vers la partie avant de la jambe 3 et une seconde extrémité étant orientée vers la partie arrière de la jambe 3. La première extrémité est destinée au montage du système d'amortissement 4. Le système d'amortissement 4 peut être monté sur cette première extrémité par une liaison pivot associée. Sur l'exemple particulier de la figure 6, la bascule comporte à sa première extrémité deux tronçons de bascule 12a, 12b séparés par un espace de vide, formant un Y. Entre les deux tronçons de bascule 12a, 12b une seconde pièce de montage 11 b (figure 6) du système d'amortissement 4 est insérée de sorte qu'un axe commun puisse être inséré dans des alésages formés respectivement au niveau des deux tronçons 12a, 12b de bascule et de la seconde pièce de montage 11 b.

La seconde extrémité de la bascule 7 est destinée au montage de la biellette 8. La biellette 8 peut être montée sur cette seconde extrémité par un axe formant une liaison pivot. Autrement dit, la bascule peut comporter à sa seconde extrémité un alésage recevant un axe sur lequel peut être monté la biellette 8.

La biellette 8, à l'opposée de son montage sur la bascule 7, peut être montée à pivotement autour d'un axe disposé à l'arrière (face arrière de la prothèse) de l'élément de support 1, ledit axe pouvant être fixe par rapport à l'élément de support 1. Sur les figures 4 et 5, la biellette 8 peut comporter deux extrémités distales conformées en Y de sorte à recevoir dans l'espace séparant les deux ramifications du Y respectivement une partie de la bascule 7 et une partie de l'élément de support 1, permettant ainsi de réaliser le montage à l'aide d'axes correspondants. Les parties de bascule et de l'élément de support 1 comportent chacune un alésage destiné à recevoir un axe formant un point de montage de la biellette 8.

Sur l'exemple particulier des figures 4 et 5, pour la biellette 8, l'axe au niveau de la bascule est fixe par rapport à la biellette 8, permettant un pivotement de la bascule autour de cet axe. L'axe au niveau de l'élément de support 1 est fixe par rapport à l'élément de support 1, permettant un pivotement de la biellette 8 autour de cet axe. Pour le système d'amortissement, il est monté à pivotement sur l'axe de la liaison pivot 2 et sur l'axe servant de point de montage sur la bascule, ledit axe de montage étant fixe par rapport à la bascule.

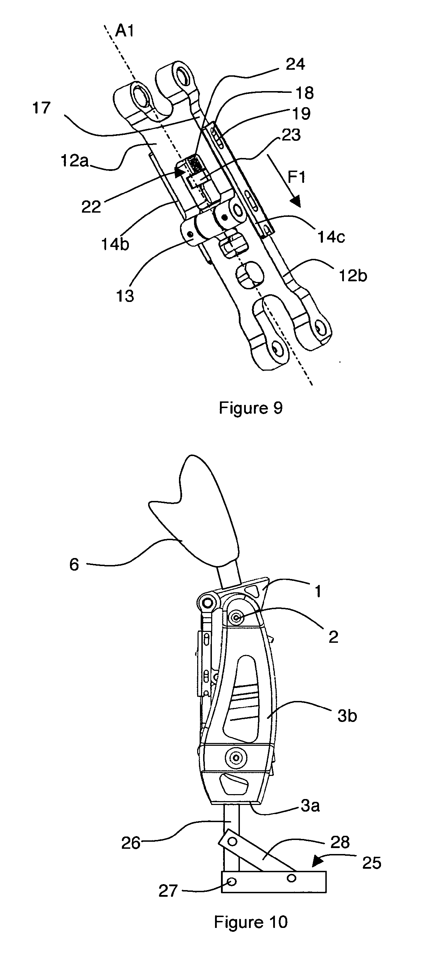

La biellette 8 peut, selon un mode de réalisation particulier, être agencée pour occuper une première position dans laquelle un mouvement de pivotement de l'élément de support 1 par rapport à la jambe 3 est transmis à la bascule 7, et une seconde position (figure 8) dans laquelle ledit mouvement n'est pas transmis à la bascule 7 (figures 4 et 5). Ceci peut, par exemple, être réalisé par une biellette 8 comme celle illustré à la figure 9, et comportant une première plaque 12a (ou élément) montée sur l'élément de support 1 (figures 4 à 8) et une seconde plaque 12b (ou élément) montée sur la bascule 7. La première plaque 12a forme charnière 13 avec la seconde plaque 12b. Autrement dit, la première plaque 12a est montée à pivotement sur la seconde plaque 12b pour éviter, le cas échéant, la transmission d'un mouvement de l'élément de support 1 à la bascule 7. La charnière 13 peut être réalisée sur la médiane de la biellette 8, perpendiculairement à l'axe longitudinal A1 de ladite biellette 8.

Cette biellette 8 à deux positions présente un avantage lorsque la prothèse est utilisée pour faire du ski. En effet, dans la première position, le skieur peut solliciter la prothèse lors d'une descente de piste, et lors de l'utilisation d'un télésiège, la prothèse peut passer en seconde position de la biellette 8 permettant au skieur de s'asseoir sans difficulté sur le siège du télésiège étant donné que le système d'amortissement 4 est désactivé.

De préférence, la biellette 8 comporte des moyens de verrouillage disposés pour empêcher le pivotement de la première plaque 12a par rapport à la seconde plaque 12b dans la première position, et pour permettre ledit pivotement dans la seconde position.

Les figures 5 à 9 illustrent un mode de réalisation particulier de tels moyens de verrouillage. Ces derniers peuvent comporter une platine 14 montée à coulissement sur l'une des plaques 12a, 12b, de préférence sur la face arrière de la prothèse, dans un plan parallèle au plan de la plaque associée. La platine 14 comporte, de préférence à une extrémité, des premiers moyens d'emboîtement 15a coopérant avec des seconds moyens d'emboîtement 15b de l'autre plaque pour verrouiller, en première position, les première et seconde plaques entre elles. Dans la première position, les première et seconde plaques sont privées de mouvements relatifs l'une par rapport à l'autre. Autrement dit, les deux plaques se comportent comme une pièce monobloc. Dans la seconde position, la première plaque 12a peut pivoter par rapport à la seconde plaque 12b, par exemple au niveau de la charnière 13, de sorte à ne pas transmettre le mouvement de l'élément de support 1 à la bascule 7, ceci permettant de relâcher les contraintes imposées au système d'amortissement 4.

Sur l'exemple particulier des figures 4 à 9, la platine 14 comporte une base 14a reliant deux flancs 14b, 14c, opposés, parallèles, et orientés selon la direction longitudinale (axe A1 sur les figures 7 et 9) de la biellette 8 en première position. La dimension transversale de la base 14a est supérieure à la dimension de transversale de la première plaque 12a de sorte que la base 14a vienne épouser une face principale 16 de ladite première plaque 12a. Dans l'exemple, la face principale 16 est sensiblement parallèle au plan de la première plaque 12a. Les deux flancs 14b, 14c opposés viennent chacun respectivement épouser un rebord 17 de la première plaque 12a (rebord sensiblement perpendiculaire à la face 16). Les rebords 17 de la première plaque 12a comportent chacun au moins une saillie 18 sensiblement perpendiculaire audit rebord 17. Chaque saillie 18 coopère avec une lumière 19 pratiquée dans le flanc 14b, 14c correspondant de la platine 14. Selon l'exemple particulier, chaque rebord 17 comporte deux saillies et chaque flanc comporte deux lumières correspondantes. Les lumières et les saillies permettent d'une part de maintenir la platine montée sur la première plaque 12a, et d'autre part de limiter la course de la platine 14 par rapport à la première plaque 12a. Autrement dit, une saillie traverse au moins en partie la lumière associée pour former une butée de déplacement de la platine 14 par rapport à la première plaque. Les dimensions transversales de la saillie sont sensiblement égales aux dimensions transversales de la lumière perpendiculairement à l'axe longitudinal des flancs.

Afin de réaliser le blocage de la première plaque 12a avec la seconde plaque 12b, la platine 14 peut comporter à une extrémité orientée vers la seconde plaque 12b deux saignées 20a, 20b (figure 8), formant les premier moyens d'emboîtement, chaque saignée étant réalisée au niveau d'un flanc de la platine 14, et orientée selon l'axe longitudinal de la platine 14. En première position, chaque saignée 20a, 20b coopère avec un ergot 21 correspondant de la seconde plaque 12b qui vient alors se loger dans la saignée associée. L'ergot 21, formant les seconds moyens d'emboîtement, est formé en saillie d'un rebord de la seconde plaque 12b.

De préférence, la platine 14 est constamment sollicitée en direction de la plaque sur laquelle elle n'est pas montée, par exemple par un organe de sollicitation tel qu'un ressort. Cette réalisation peut être mise en oeuvre comme, par exemple, à la figure 9 sur laquelle la première plaque 12a comporte une ouverture 22 débouchante pratiquée au niveau d'une de ses faces perpendiculairement au plan de ladite première plaque 12a. La base de la platine comporte alors entre les deux flancs 14b, 14c une protubérance 23 apte à se déplacer dans l'ouverture 22, ladite protubérance 23 pouvant alors être reliée à une face intérieure de l'ouverture par un ressort 24 de sorte à solliciter le déplacement de la platine 14 dans une direction opposée au premier élément (selon la flèche F1 à la figure 8). Sur la figure 8, le ressort 24 est un ressort de compression, et ce dernier relie la protubérance 23 à une face intérieure de l'ouverture distale de la charnière 13. Bien entendu, l'homme du métier peut aussi utiliser un ressort de traction solidarisé à la protubérance et à une face intérieure de l'ouverture proximale de la charnière 13.

Ainsi, l'utilisateur de la prothèse peut tirer sur la platine dans une direction opposée à la flèche F1 pour désemboîter les saignées de leurs ergots respectifs, et passer la prothèse en seconde position de la biellette 8. Afin de permettre un retour à la première position, l'utilisateur peut réaliser l'action inverse. De manière préférentielle, les extrémités des flancs 14b, 14c de la platine 14 au niveau des saignées, et en regard des ergots associés, comportent chacune un chanfrein permettant un verrouillage automatique des saignées avec les ergots lorsque l'utilisateur reprend une position debout. En effet, lors du retour à la position debout, la biellette 8 va avoir tendance à revenir en première position et le chanfrein vient alors en contact avec un ergot associé, repoussant naturellement la platine 14 dans une direction opposée à la flèche F1 jusqu'à l'emboîtement. Autrement dit, de manière générale, les premiers et seconds moyens d'emboîtement peuvent être conformés pour s'emboîter automatiquement lors d'une extension de la prothèse lorsque cette dernière est en seconde position.

Bien entendu, il s'agit d'un exemple de réalisation, l'homme du métier sera à même de modifier l'assemblage, par exemple en inversant le montage de la première plaque et de la seconde plaque respectivement sur la bascule et sur l'élément de support.

Selon un développement, le système d'amortissement comporte un vérin. Le vérin est, de préférence, de type oléopneumatique. Il permet, de préférence un réglage distinct de sa force de compression et de sa vitesse de détente. Autrement dit, le vérin peut comporter des moyens distincts de réglage de sa force de compression et de sa vitesse de détente. Par exemple la vitesse de détente peut être réglée pour permettre un retour plus doux de la position en flexion vers la position en extension. Ceci permet notamment de régler le membre inférieur en fonction du niveau et du désir de l'utilisateur.

Par extension, en parlant de la prothèse d'un membre inférieur, l'organe de réception d'un moignon de cuisse, l'élément de support et la jambe sont des éléments mécaniques permettant à une personne amputée, par exemple un skieur, de retrouver les deux appuis des membres inférieurs, et de réaliser des fonctions proches de celles d'un vrai membre inférieur dans des conditions particulières comme la position en triple flexion (hanche, genou, cheville). Pour cela, les axes évoqués ci-dessus (axe de la liaison pivot, les axes de montage de la biellette sur l'élément de support et sur la bascule, l'axe de montage du système d'amortissement sur la bascule et l'axe de basculement de la bascule), sont tous, de préférence, sensiblement parallèles entre eux.

Comme illustré à la figure 10, la prothèse peut en outre comporter un pied 25 monté à une extrémité de la jambe 3 opposée à l'élément de support 1, par exemple sur une tige 26 de la jambe fixée au corps principal 3a, 3b, 3c à l'opposé de l'élément de support. Le pied 25 est monté à pivotement sur ladite jambe 3, l'axe de pivotement est alors sensiblement parallèle à l'axe de la liaison pivot 2 formant l'articulation. La tige 26 peut être montée sur le pied 25 par un axe 27 au niveau de la zone appelée cou-de-pied. La tige 26 peut être reliée par l'intermédiaire d'un vérin 28 avec une extrémité du pied 25 opposée à la tige 26. L'utilisation du vérin 28 au niveau du pied permet d'obtenir une compensation de la flexion de la prothèse au niveau de l'articulation par une flexion dorsale du pied.

Selon une variante (non représentée), une semelle est montée sur une face inférieure du pied, ladite semelle adoptant une forme destinée à coopérer avec une fixation pour chaussure de ski.

Dans le cas particulier des sports de neige, l'amplitude de mouvement apportée par une telle prothèse permet de s'adapter à tout type de pratique de ski ou de surf.

L'élément de support 1 peut comporter à l'opposé de l'articulation (liaison pivot 2) une surface, de préférence, sensiblement plane destinée à fixer l'organe de réception 6 d'un moignon de cuisse. Cet organe de réception peut comporter une plaque, par exemple fixée à ladite surface par des vis. La plaque de l'organe de réception est elle-même fixée à un réceptacle, par exemple formé en fibre de carbone, conformé selon le moignon du membre inférieur de l'utilisateur.

Des tests de cette prothèse utilisant un vérin comme système d'amortissement, ont permis de démontrer qu'avec l'angle α de flexion de 155 degrés, la force exercée par le vérin est d'environ 2200N Newton alors qu'avec un angle de flexion α de 110 degrés la force exercée par le vérin du système d'amortissement Fverin est d'environ 3340N. Autrement dit, par rapport à l'art antérieur, la prothèse décrite ci-dessus permet de conserver une force d'amortissement sensiblement constante sur le vérin quel que soit l'angle de flexion au genou, notamment grâce à l'utilisation d'une force de renvoi. Ainsi, contrairement à l'art antérieur, cela évite de perdre l'amortissement et procure un meilleur confort d'utilisation se rapprochant de celui d'un membre inférieur valide.

Outre que la prothèse soit plus robuste, le fait de garder au niveau du système d'amortissement une force sensiblement constante quel que soit l'angle α de flexion de la prothèse permet de conserver des performances accrues tout au long d'une descente à ski. Ceci permet notamment de prendre appui sur la prothèse en conservant un contrôle élevé du ski même en appui unilatéral.

## Revendications

1. Prothèse pour membre inférieur comportant:
- un organe de réception (6) d'un moignon de cuisse monté sur un élément de support (1),
- une jambe (3) montée sur ledit élément de support (1) par une liaison pivot (2) formant une articulation de genou pour permettre une flexion ou une extension de la jambe (3) par rapport à l'organe de réception (6),
- un système d'amortissement (4) pour amortir les efforts de flexion ou d'extension,
prothèse **caractérisée en ce que** le système d'amortissement (4) est monté d'une part sur l'axe de la liaison pivot (2), et d'autre part sur une première extrémité d'une bascule (7) montée à pivotement sur la jambe (3), et **en ce**
**qu'**une biellette (8) est montée d'une part sur une seconde extrémité de la bascule (7) et d'autre part sur l'élément de support (1), et **en ce que** les points de montage respectifs du système d'amortissement (4) et de la biellette (8) au niveau de la bascule sont disposées de part et d'autre de l'axe de basculement (9) de ladite bascule (7).

2. Prothèse selon la revendication 1, **caractérisée en ce que** la biellette (8) est agencée pour occuper une première position dans laquelle un mouvement de pivotement de l'élément de support (1) par rapport à la jambe (3) est transmis à la bascule (7), et une seconde position dans laquelle ledit mouvement n'est pas transmis à la bascule (7).

3. Prothèse selon la revendication 2, **caractérisée en ce que** la biellette (8) comporte une première plaque (12a) montée sur l'élément de support (1), une seconde plaque (12b) montée sur la bascule (7), ladite première plaque (12a) formant charnière (13) avec la seconde plaque (12b), et **en ce que** la biellette (8) comporte des moyens de verrouillage disposés pour empêcher le pivotement de la première plaque (12a) par rapport à la seconde plaque (12b) dans la première position, et pour permettre ledit pivotement dans la seconde position.

4. Prothèse selon la revendication 3, **caractérisée en ce que** les moyens de verrouillage comportent une platine (14) montée à coulissement sur l'une des plaques (12a, 12b), ladite platine (14) comportant des premiers moyens d'emboîtement (15a) coopérant avec des seconds moyens d'emboîtement (15b) de l'autre plaque (12a, 12b) pour verrouiller, en première position, les première et seconde plaques (12a, 12b) entre elles.

5. Prothèse selon la revendication 4, **caractérisée en ce que** la platine (14) est constamment sollicitée en direction de ladite autre plaque (12a, 12b) par un organe de sollicitation.

6. Prothèse selon l'une des revendications 1 à 5 **caractérisé en ce que** le système d'amortissement (4) comporte un vérin.

7. Prothèse selon l'une quelconque des revendications 1 à 6, **caractérisée en ce qu'**elle comporte un pied monté à une extrémité de la jambe (3), opposée à l'élément de support (1), ledit pied étant monté à pivotement sur ladite jambe (3), l'axe de pivotement étant sensiblement parallèle à l'axe de la liaison pivot (2) formant l'articulation.

8. Prothèse selon la revendication 7, **caractérisée en ce qu'**une semelle est montée sur une face inférieure du pied, ladite semelle adoptant une forme destinée à coopérer avec une fixation pour chaussure de ski.

## Patentansprüche

1. Prothese für untere Gliedmaßen, enthaltend:
- ein Aufnahmeglied (6) zum Aufnehmen eines Oberschenkelstumpfs, das an einem Tragelement (1) montiert ist,
- einen Unterschenkel (3), der an dem Tragelement (1) über eine Schwenkverbindung (2) gelagert ist, die ein Kniegelenk bildet, um eine Beugung bzw. eine Streckung des Unterschenkels (3) bezüglich des Aufnahmeglieds (6) zu gestatten,
- ein Dämpfungssystem (4), um die Beuge- bzw. Streckkräfte abzudämpfen,
**dadurch gekennzeichnet, dass** das Dämpfungssystem (4) einerseits an der Achse der Schwenkverbindung (2) und andererseits an einem ersten Ende einer Wippe (7) montiert ist, die verschwenkbar am Unterschenkel (3) gelagert ist,
und dass ein Schwenkarm (8) einerseits an einem zweiten Ende der Wippe (7) und anderseits an dem Tragelement (1) montiert ist, und dass die jeweiligen Lagerungspunkte des Dämpfungssystems (4) und des Schwenkarms (8) im Bereich der Wippe auf beiden Seiten der Schwenkachse (9) der Wippe (7) angeordnet sind.

2. Prothese nach Anspruch 1, **dadurch gekennzeichnet, dass** der Schwenkarm (8) dazu vorgesehen ist, eine erste Stellung einzunehmen, in welcher eine Schwenkbewegung des Tragelements (1) bezüglich des Unterschenkels (3) auf die Wippe (7) übertagen wird, sowie eine zweite Stellung, in welcher die Bewegung nicht auf die Wippe (7) übertragen wird.

3. Prothese nach Anspruch 2, **dadurch gekennzeichnet, dass** der Schwenkarm (8) eine erste Platte (12a) aufweist, die am Tragelement (1) montiert ist, sowie eine zweite Platte (12b), die an der Wippe (7) montiert ist, wobei die erste Platte (12a) ein Scharnier (13) mit der zweiten Platte (12b) bildet, und dass der Schwenkarm (8) Verriegelungsmittel aufweist, die dazu vorgesehen sind, das Verschwenken der ersten Platte (12a) gegenüber der zweiten Platte (12b) in der ersten Stellung zu verhindern und um das Verschwenken in der zweiten Stellung zu gestatten.

4. Prothese nach Anspruch 3, **dadurch gekennzeichnet, dass** die Verriegelungsmittel eine Platine (14) aufweisen, die an einer der Platten (12a, 12b) gleitbeweglich gelagert ist, wobei die Platine (14) erste Einrastmittel (15a) aufweist, die mit zweiten Einrastmitteln (15b) der anderen Platte (12a, 12b) zusammenwirken, um in der ersten Stellung die erste und die zweite Platte (12a, 12b) miteinander zu verriegeln.

5. Prothese nach Anspruch 4, **dadurch gekennzeichnet, dass** die Platine (14) stets von einem Beaufschlagungsglied in Richtung der anderen Platte (12a, 12b) beaufschlagt wird.

6. Prothese nach einem der Ansprüche 1 bis 5, **dadurch gekennzeichnet, dass** das Dämpfungssystem (4) einen Kraftzylinder aufweist.

7. Prothese nach einem der Ansprüche 1 bis 6, **dadurch gekennzeichnet, dass** sie einen Fuß aufweist, der an einem dem Tragelement (1) entgegengesetzten Ende des Unterschenkels (3) montiert ist, wobei der Fuß am Unterschenkel (3) verschwenkbar gelagert ist, wobei die Schwenkachse im Wesentlichen parallel zur Achse der das Gelenk (2) bildenden Schwenkverbindung (2) verläuft.

8. Prothese nach Anspruch 7, **dadurch gekennzeichnet, dass** eine Sohle an einer Unterseite des Fußes montiert ist, wobei die Sohle eine Form annimmt, die dazu bestimmt ist, mit einer Skibindung zusammenzuwirken.

## Claims

1. Lower limb prosthesis having:
- a receiving member (6) provided for receiving a thigh stump and mounted on a support element (1),
- a leg (3) mounted on said support element (1) via a pivot link (2) forming a knee joint in order to permit flexion or extension of the leg (3) with respect to the receiving member (6),
- a damping system (4) for damping the flexion or extension forces,
said prosthesis being **characterized in that** the damping system (4) is mounted, on the one hand, on the axis of the pivot link (2) and, on the other hand, on a first end of a rocker (7) that is mounted pivotably on the leg (3), and **in that** a connecting rod (8) is mounted, on the one hand, on a second end of the rocker (7) and, on the other hand, on the support element (1), and **in that** the respective mounting points of the damping system (4) and of the connecting rod (8) at the level of the rocker are arranged on either side of the tilt axis (9) of said rocker (7).

2. Prosthesis according to Claim 1, **characterized in that** the connecting rod (8) is designed to occupy a first position, in which a pivoting movement of the support element (1) with respect to the leg (3) is transmitted to the rocker (7), and a second position, in which said movement is not transmitted to the rocker (7).

3. Prosthesis according to Claim 2, **characterized in that** the connecting rod (8) has a first plate (12a) mounted on the support element (1), and a second plate (12b) mounted on the rocker (7), said first plate (12a) forming a hinge (13) with the second plate (12b), and **in that** the connecting rod (8) has locking means arranged so as to prevent the pivoting of the first plate (12a) with respect to the second plate (12b) in the first position and to permit said pivoting in the second position.

4. Prosthesis according to Claim 3, **characterized in that** the locking means comprise a mounting plate (14) mounted slidably on one of the plates (12a, 12b), said mounting plate (14) having first engagement means (15a) cooperating with second engagement means (15b) of the other plate (12a, 12b) in order to lock the first and second plates (12a, 12b) to each other in the first position.

5. Prosthesis according to Claim 4, **characterized in that** the mounting plate (14) is constantly biased in the direction of said other plate (12a, 12b) by a biasing member.

6. Prosthesis according to any of Claims 1 to 5, **characterized in that** the damping system (4) has a jack.

7. Prosthesis according to any of Claims 1 to 6, **characterized in that** it has a foot mounted at one end of the leg (3), opposite the support element (1), said foot being mounted pivotably on said leg (3), the axis of pivoting being substantially parallel to the axis of the pivot link (2) forming the joint.

8. Prosthesis according to Claim 7, **characterized in that** a sole is mounted on a bottom surface of the foot, said sole adopting a shape intended to cooperate with a binding for a ski boot.
